# EUROPEAN PATENT APPLICATION

(11) **EP 1 362 927 A1**
(43) Date of publication of application: **19.11.2003**
(21) Application number: 03010897.1
(22) Date of filing: 15.05.2003
(51) Int. Cl.: C12Q 1/68, C12Q 1/70

(54) **Nucleic acid recovery reagents and methods**

(30) Priority: 17.05.2002 US 150405
(71) Applicant: MAYO FOUNDATION FOR MEDICAL EDUCATION AND RESEARCH, Rochester, MN 55905 (US)
(72) Inventor: Cockerill, Franklin R. III, Rochester, Minnesota 55905 (US); Rosenblatt, Jon E., Rochester, Minnesota 55906 (US); Sloan, Lynne M., Rochester, Minnesota 55902 (US); Uhl, James R., Rochester, Minnesota 55902 (US)
(74) Representative: Gassner, Wolfgang, Dr.

(57) **Abstract**

Disclosed are reagents and techniques useful for recovering nucleic acids from stool samples. The recovered nucleic acids can be used in medical and veterinary diagnostic procedures that involve nucleic acid-based detection of pathogens and tumor cells.

## Description

### TECHNICAL FIELD

This invention relates to compositions useful for stabilizing nucleic acids in biological samples.

### BACKGROUND

Many medical and veterinary diagnostic procedures involve obtaining a biological sample from a patient and analyzing nucleic acids present therein (e.g., to determine the presence or identity of infectious organisms or tumor cells). Problematically, nucleases can degrade nucleic acids in biological samples (e.g., after collection and during analysis). Such degradation can adversely affect a clinician's ability to detect nucleic acids in a sample, and can lead to false negative diagnoses. Thus, to preserve the integrity of nucleic acids in collected samples, many commercially available sample processing reagents and tools are rid of nucleases and / or supplemented with nuclease inhibitors.

Clinicians often look to stool samples to diagnose enteric diseases, such as those caused by *Salmonella* spp., *Vibrio* spp., *Campylobacter jejuni*, and *E. Coli* (e.g., serotype O157:H7). To detect such pathogens, clinicians typically employ bacterial culture and immunological approaches. Such approaches can be time and resource intensive. Enzymatic amplification-based analysis of nucleic acids in stool samples could accelerate therapeutic intervention and improve patient outcomes. Presently available materials and methods have not proven completely satisfactory for sensitive and reliable enzymatic amplification and analysis of nucleic acids from stool samples in a clinical setting.

### SUMMARY

The invention is based, in part, on the surprising discovery that stool samples contain large amounts of nucleases. Current nucleic acid recovery materials and methods typically leave nucleic acids in stool samples vulnerable to degradation by these nucleases during sample collection and analysis.

The invention features new reagents and techniques that can facilitate the recovery of nucleic acids from stool samples. Recovered nucleic acids can be used for a variety of diagnostic purposes, including nucleic acid-based testing for a variety of pathogens and tumor cells.

In one aspect, the invention features methods for stabilizing nucleic acids in a stool sample. In some embodiments, the sample is derived from a mammal (e.g., non-human primate, equine, bovine, canine, feline, porcine and human). The methods involve mixing a stool sample with a nucleic acid recovery reagent to yield a sample-reagent mixture, wherein the reagent comprises a detergent and a cation chelator. In some embodiments, the ratio of sample to reagent is 1:10 to 1:1 sample to reagent. In some embodiments, 1 to 25 ml of sample is mixed with reagent. The methods additionally can involve separating nucleic acids from bulk sample material. In some embodiments, chloroform is included in the sample-reagent mixture (e.g., at a ratio of 1:100 to 1:5 chloroform to mixture). In some embodiments, the reagent includes a detergent (e.g., ammonium lauryl sulfate, sodium lauryl sulfate and sodium laureth sulfate) at 0.05% to 0.5%. In some embodiments, the reagent includes a cation chelator (e.g., EDTA, EGTA and citrate) at 0.05M to 0.2M. in some embodiments, the pH of the reagent is 4.0 to 7.0.

In another aspect, the invention features methods for detecting a diagnostic nucleic acid (e.g., DNA and RNA) in a stool sample. In some embodiments the diagnostic nucleic acid is derived from an infectious agent such as a bacteria (e.g., *Helicobacter pylori*, Verotoxin-producing *E. coli, Shigella* spp., *Salmonella* spp., *Campylobacter jejuni, Clostridium difficile, Yersinia enterocolitica* and *Bacillus anthracis*), virus (e.g., enterovirus, herpes virus, rotavirus, norwalk virus and adenovirus), or protozoa (microsporidia, cryptosporidium, *giardia lamblia* and *Entamoeba histolytica*). In some embodiments, the diagnostic nucleic acid is an aerodigestive tract tumor marker (e.g., P16, P14, MGMT, MLH1 P15, APC, kRAS, IFG2 and MYOD1 nucleic acid). In some embodiments, the sample is derived from a mammal (e.g., non-human primate, equine, bovine, canine, feline, porcine and human). The methods involve: 1) providing a sample-reagent mixture comprising a stool sample mixed with a nucleic acid recovery reagent; 2) separating the diagnostic nucleic acid from bulk stool materials; and 3) detecting the presence or absence of said nucleic acid. The reagent comprises a detergent and a cation chelator. In some embodiments, the reagent includes a detergent (e.g., ammonium lauryl sulfate, sodium lauryl sulfate and sodium laureth sulfate) at 0.05% to 0.5%. In some embodiments, the reagent includes a cation chelator (e.g., EDTA, EGTA and citrate) at 0.05M to 0.2M. In some embodiments, the pH of the reagent is 4.0 to 7.0. In some embodiments, the ratio of sample to reagent is 1:10 to 1:1 sample to reagent. In some embodiments, 1 to 25 ml of sample is mixed with reagent. In some embodiments, chloroform is included in the sample-reagent mixture (e.g., at a ratio of 1:100 to 1:5 chloroform to mixture).

In another aspect, the invention features kits for recovering nucleic acids from a stool sample. The kits include a contained nucleic acid recovery reagent and instructions indicating how the reagent can be mixed with a stool sample, wherein the reagent comprises a detergent and a cation chelator. In some embodiments, the kits include a stool sample collection instrument and a stool sample containment vessel. In some embodiments, the reagent is included in a unit-use volume of 5 ml to 50 ml (e.g., in a stool sample containment vessel). In some embodiments, the kits include contained chloroform (e.g. in a stool sample containment vessel).

Other features and advantages of the invention will be apparent from the following detailed description, and from the claims. Unless otherwise defined, all technical and scientific terms used herein have the meaning commonly understood by one of ordinary skill in the art to which this invention belongs. All publications, patent applications, patents, and other references mentioned herein are incorporated by reference in their entirety. In case of conflict, the present specification, including definitions, will control. The disclosed materials, methods, and examples are illustrative only and not intended to be limiting. Skilled artisans will appreciate that methods and materials similar or equivalent to those described herein can be used to practice the invention.

### DETAILED DESCRIPTION

The invention provides reagents and techniques useful for stabilization and recovery of nucleic acids from stool samples. The reagents and techniques can be applied to stool samples obtained from any organism, including mammals such as humans, non-human primates, bovines, equines, porcines, canines and felines. Recovered nucleic acids can be used in medical and veterinary diagnostic procedures that involve nucleic acid-based detection of pathogens and tumor cells.

### Nucleic Acid Recovery Reagents and Kits

Nucleic acid recovery reagents in accord with the invention include one or more detergents and one or more cation chelators. Detergents facilitate the extraction of nucleic acids from bulk stool material and prevent the carryover of substances that can inhibit enzymatic nucleic acid amplification (e.g., by the polymerase chain reaction). Suitable detergents include ammonium lauryl sulfate, sodium lauryl sulfate and sodium laureth sulfate. In some embodiments, a detergent is included in a nucleic acid recovery reagent in an amount from about 0.05% to 0.5%. Cation chelators inhibit nucleases, which are surprisingly abundant in stool samples. Most of these nucleases appear to require divalent cations such as Mg²⁺, Mn²⁺ and Ca²⁺ to catalytically degrade nucleic acids. Cation chelators can make such cations unavailable to nucleases. Suitable cation chelators include ethylenediaminetetraacetic acid (EDTA), ethylene glycol-bis(2-aminoethylether)-N,N,N',N'-tetraacetic acid (EGTA) and citrate. In some embodiments, a cation chelator is included in a nucleic acid recovery reagent in an amount from about 0.05M to 0.2M. Relatively high concentrations of detergents (e.g., 0.2% to 0.5%) and cation chelators (e.g., 0.1M to 0.2M) can minimize dilution of stool samples and can improve the sensitivity of nucleic acid-based diagnostic techniques. Nucleic acid recovery reagents typically have a pH of about 4 to 7 (e.g., 4 to 5, 5 to 6, and 4 to 6). One of skill in the art can, as a matter of routine experimentation, adjust the amount and identity of the detergent(s) and cation chelator(s) in a nucleic acid recovery reagent to optimize the reagent formulation for particular clinical applications.

Nucleic acid recovery reagents can be combined with packaging material and sold as a kit. A nucleic acid recovery kit typically includes instructions that describe how the reagent can be mixed with a stool sample. A nucleic acid recovery kit also can include a stool sample collection instrument, e.g., a spoon or spatula. In some embodiments, a nucleic acid recovery reagent is included within a stool sample collection vessel such as, or similar to, those available commercially from Globe Scientific Inc. (catalog no. 109120 and 109117), Quelab Laboratories Inc. (catalog no. EPT-100, EPT-100C, and EPT-101) and Meridian Diagnostics Inc. (catalog no. 900112, 900212, and 900312). A nucleic acid recovery reagent can be included in a sample collection vessel or in a different vessel. In some embodiments, a nucleic acid recovery reagent is packaged in a unit-use volume (i.e., for mixing with a single stool sample) of 5 ml to 50 ml (e.g., 5 ml to 10 ml, 5 ml to 25 ml, 10 ml to 25 ml, and 10 ml and 50 ml). A unit-use volume of nucleic acid recovery reagent also can include a predefined amount a known nucleic acid (e.g., as a positive control for recovery). A nucleic acid recovery kit also can include chloroform. Chloroform can be packaged in the same vessel as the nucleic acid recovery reagent or can be packaged in a different vessel.

### Nucleic Acid Recovery Methods

Nucleic acid recovery reagents can be used to recover diagnostic nucleic acids (e.g., DNA and RNA) from stool samples, including nucleic acids derived from infectious agents such as *Helicobacter pylori*, Verotoxin (Shiga-like toxin) producing *E. coli, Shigella* spp., *Salmonella* spp., *Campylobacter jejuni, Clostridium difficile, Yersinia enterocolitica, Bacillus anthracis*, enterovirus, herpes virus, rotavirus, norwalk virus, adenovirus, microsporidia, cryptosporidium, *giardia lamblia* and *Entamoeba histolytica*. Nucleic acid recovery reagents also can be used to recover aerodigestive tract tumor markers (i.e., carcinoma-associated nucleic acids that can be found the aerodigestive tract) such as P16, P14, MGMT, MLH1 P15, APC and kRAS (see e.g., Esteller, M. and Herman, J.G. (2002) *J Pathol*. 196:1-7), IFG2 (see e.g., Okamoto, K. et al. (1997) *Proc Natl Acad Sci U.S.A*. 94:5367-5371) and MYOD1 (see e.g. Folpe, A.L. *Adv Anat Pathol*. (2002) 9:198-203), nucleic acids.

Nucleic acid recovery methods in accord with the invention involve mixing a stool sample with a nucleic acid recovery reagent. Typically, about 1 ml to 25 ml (e.g., 1 ml to 2 ml, 1 ml to 5 ml, 1 ml to 10 ml, 5 to 25, and 10 ml to 25 ml) of stool sample is obtained and mixed with a nucleic acid recovery reagent. In some embodiments, the sample and reagent are combined in a ratio of about 1:10 to 1:1 (e.g., 1:10 to 1:5, 1:5 to 1:1, 1:10 to 1:3, and 1:3 to 1:1) sample to reagent. The sample reagent ratio depends on, *inter alia*, the consistency of the sample, the concentration of the detergent and cation chelator in the nucleic acid recovery reagent, and the expected duration of sample storage and analysis. One of skill in the art can, as a matter of routine, optimize the sample:reagent ratio in accord with sampling conditions and analysis protocols.

In some applications, chloroform is included in a nucleic acid recovery reagent-sample mixture. Chloroform can remove stool sample components that could inhibit nucleic acid recovery or detection. Chloroform also can kill or suspend the growth of microorganisms present in a stool sample, including *M. tuberculosis*, making samples safer for clinicians to handle. In some embodiments, choloroform is included in a sample-reagent mixture at a ratio of about 1:100 to 1:5 (e.g., 1:100 to 1:50, 1:50 to 1:20, and 1:20 to 1:5) chloroform to sample-reagent. When used, chloroform can be added to a sample, to a nucleic acid stabilization reagent, or to a sample-reagent mixture.

Nucleic acids can be separated from bulk sample material by, e.g., centrifugation or filtration, and can be further separated from sample materials using a variety of purification tools and techniques known in the art. Many useful nucleic acid purification tools are available commercially, including MagNA Pure™ Instrument and Total Nucleic Acid Kit, Catalog No. 3038505 (Roche Applied Sciences, Indianapolis, IN); QIAamp™ DNA Stool Mini Kit, Catalog No. 51504 (Qiagen, Valencia, CA); High Pure PCR Template Preparation Kit, Catalog No. 1754777 (Roche Applied Sciences, Indianapolis, IN); and IsoQuick™ Nucleic Acid Extraction Kit, Catalog No. MXT-020-100 (ORCA Research, Inc., Bothell, WA). One of skill in the art can, as a matter of routine, optimize and adapt nucleic acid separation reagents and protocols for particular diagnostic applications. Separated nucleic acids can be analyzed and detected by any means known in the art.

A stool sample mixed with a nucleic acid recovery reagent can be provided and analyzed by the one entity, or can be provided by a patient or clinical entity and delivered to another entity for analysis.

The invention will be further described in the following examples, which do not limit the scope of the invention described in the claims.

### EXAMPLES

### Example 1: Nucleic acid recovery buffer.

This example describes the preparation of a nucleic acid recovery buffer including 0.2 M Citrate, 0.2 M EDTA, and 0.5% ammonium lauryl sulfate (pH 5.0).

Dissolve 20.5 g NaOH and 74.4 g EDTA, disodium dihydrate (FW372.2) in 900 ml distilled water in a flask using a magnetic stir bar. Add 42.0 g citric acid monohydrate (FW 210.14) and continue stirring to dissolve. Adjust the pH to 4.9 - 5.1 if necessary. Add 17.8 ml ammonium lauryl sulfate (28%) and stir to dissolve. Add distilled water to bring the volume to 1000 ml. The buffer can be stored at room temperature.

### Example 2: Recovery and analysis of nucleic acids from stool samples.

This example describes protocols for recovering and analyzing diagnostic nucleic acids from stool samples. Nucleic acids from the infectious agents *E. coli* O157:H7, vancomycin-resistant *E. coli, Bacillus anthracis*, enterovirus (RNA) and herpes virus (DNA) were added to and then recovered from stool samples. For each infectious agent, 7 to 14 different stool samples were processed as follows.

Stool samples were spiked with an infectious agent at concentrations ranging from approximately 10⁶ to 10⁴ organisms per ml. Five ml of stool sample was mixed with 15 ml of nucleic acid recovery reagent (0.2 M Citrate, 0.2 M EDTA and 0.5% ammonium lauryl sulfate (pH 5.0)) and 2 ml of chloroform in a polypropylene tube. Sample-reagent-chloroform mixtures were vortexed 10 seconds and centrifuged at 1000 x g for 1 min. Control samples were mixed with water instead of nucleic acid recovery reagent.

Nucleic acids were extracted from 200 ul aliquots of the resultant supernatant using a MagNA Pure instrument and Total Nucleic Acid Kit, Catalog No. 3038505 (Roche Applied Sciences, Indianapolis, IN). A LightCycler™ assay was used to evaluate recovery of diagnostic nucleic acid (e.g., Shiga toxin DNA for samples spiked with *E. coli* O157:H7). A detailed description of the LightCycler™ System and real-time and on-line monitoring of PCR can be found at http://biochem.roche.com/lightcycler. In addition, the following patent applications describe real-time PCR as used in the LightCycler™ technology: WO 97/46707, WO 97/46714 and WO 97/46712. The diagnostic DNA was recovered from most samples (e.g., 95% of samples spiked with *E. coli* O157:H7) that were mixed with the nucleic acid recovery reagent, and was not recovered from most control samples. Diagnostic nucleic acids also were detected in mixed samples that were stored at room temperature for 1, 3 and 7 days before processing.

### OTHER EMBODIMENTS

It is to be understood that while the invention has been described in conjunction with the detailed description thereof, the foregoing description is intended to illustrate and not limit the scope of the invention.

## Claims

1. A method for stabilizing nucleic acids in a stool sample, said method comprising mixing a stool sample with a nucleic acid recovery reagent to yield a sample-reagent mixture, wherein said reagent comprises a detergent and a cation chelator.

2. The method of claim 1, further comprising separating said nucleic acids from bulk sample material.

3. The method of claim 1, wherein said sample is derived from a mammal.

4. The method of claim 3, wherein said mammal is selected from the group consisting of non-human primate, equine, bovine, canine, feline and porcine.

5. The method of claim 3, wherein said mammal is human.

6. The method of claim 1, further comprising including chloroform in said sample-reagent mixture.

7. The method of claim 6, wherein the ratio of chloroform to said sample-reagent mixture is 1:100 to 1:5 chloroform to mixture.

8. The method of claim 1, wherein said detergent is selected from the group consisting of ammonium lauryl sulfate, sodium lauryl sulfate and sodium laureth sulfate.

9. The method of claim 8, wherein the amount of said detergent in said reagent is 0.05% to 0.5%.

10. The method of claim 1, wherein said cation chelator is selected from the group consisting of EDTA, EGTA and citrate.

11. The method of claim 10, wherein the amount of said cation chelator in said reagent is 0.05M to 0.2M.

12. The method of claim 1, wherein the ratio of said sample to said reagent is 1:10 to 1:1 sample to reagent.

13. The method of claim 1, wherein the pH of said reagent is 4.0 to 7.0.

14. The method of claim 1, wherein 1 to 25 ml of said sample is mixed with said reagent.

15. A method for detecting a diagnostic nucleic acid in a stool sample, said method comprising:
a. providing a sample-reagent mixture comprising a stool sample mixed with a nucleic acid recovery reagent, said reagent comprising a detergent and a cation chelator;
b. separating said nucleic acid from bulk stool materials, and
c. detecting the presence or absence of said nucleic acid.

16. The method of claim 15, wherein said nucleic acid is derived from an infectious agent.

17. The method of claim 16, wherein said nucleic acid is RNA.

18. The method of claim 16, wherein said nucleic acid is DNA.

19. The method of claim 16, wherein said infectious agent is a bacteria.

20. The method of claim 19, wherein said bacteria is selected from the group consisting of *Helicobacter pylori*, Verotoxin-producing *E. coli, Shigella* spp., *Salmonella* spp., *Campylobacter jejuni, Clostridium difficile, Yersinia enterocolitica* and *Bacillus anthracis*.

21. The method of claim 16, wherein said infectious agent is a virus.

22. The method of claim 21, wherein said virus is selected from the group consisting of enterovirus, herpes virus, rotavirus, norwalk virus and adenovirus.

23. The method of claim 16, wherein said infectious agent is a protozoa.

24. The method of claim 23, wherein said protozoa is selected from the group consisting of microsporidia, cryptosporidium, *giardia lamblia* and *Entamoeba histolytica*.

25. The method of claim 15, wherein said nucleic acid is an aerodigestive tract tumor marker.

26. The method of claim 25, wherein said nucleic acid is RNA.

27. The method of claim 25, wherein said nucleic acid is DNA.

28. The method of claim 25, wherein said tumor marker is selected from the group consisting of P16, P14, MGMT, MLH1 P15, APC, kRAS, IFG2 and MYOD1 nucleic acid.

29. The method of claim 15, wherein said sample is derived from a mammal.

30. The method of claim 29, wherein said mammal is selected from the group consisting of non-human primate, equine, bovine, canine, feline and porcine.

31. The method of claim 29, wherein said mammal is human.

32. The method of claim 15, wherein said sample-reagent mixture contains chloroform.

33. The method of claim 32, wherein the ratio of chloroform to said sample-reagent mixture is 1:100 to 1:5 chloroform to mixture.

34. The method of claim 15, wherein said detergent is selected from the group consisting of ammonium lauryl sulfate, sodium lauryl sulfate and sodium laureth sulfate.

35. The method of claim 34, wherein the amount of said detergent in said reagent is 0.05% to 0.5%.

36. The method of claim 15, wherein said cation chelator is selected from the group consisting of EDTA, EGTA and citrate.

37. The method of claim 36, wherein the amount of said cation chelator in said reagent is 0.05M to 0.2M.

38. The method of claim 15, wherein the ratio of said sample to said nucleic acid recovery reagent is 1:10 to 1:1 sample to reagent.

39. The method of claim 15, wherein the pH of said reagent is 4.0 to 7.0.

40. The method of claim 15, wherein said sample-reagent mixture includes 1 ml to 25 ml of stool material.

41. A kit for recovering nucleic acids from a stool sample, said kit comprising a contained nucleic acid recovery reagent, and instructions indicating how said reagent can be mixed with said stool sample, wherein said reagent comprises a detergent and a cation chelator

42. The kit of claim 41, further comprising a stool sample collection instrument and a stool sample containment vessel.

43. The kit of claim 41, wherein said reagent is included in a unit-use volume of 5 ml to 50 ml.

44. The kit of claim 43, wherein said reagent is contained in a stool sample containment vessel.

45. The kit of claim 41, further comprising contained chloroform.

46. The kit of claim 45, wherein said chloroform is contained in a stool sample containment vessel.
